# EUROPÄISCHE PATENTANMELDUNG

(11) **EP 3 266 760 A1**
(43) Veröffentlichungstag der Anmeldung: **10.01.2018**
(21) Anmeldenummer: 16178408.7
(22) Anmeldetag: 07.07.2016
(51) Int. Cl.: C07C 41/18

(54) **VERFAHREN ZUR HERSTELLUNG VON VINYLALKYLENGLYKOLETHERN**

(71) Anmelder: BASF SE, 67056 Ludwigshafen am Rhein (DE)
(72) Erfinder: ERNST, Martin, 67056 Ludwigshafen (DE); WEICHMANN, Josef, 83308 Trostberg (DE); BURK, Simon, 67056 Ludwigshafen (DE); D'ANDOLA, Giovanni, 67056 Ludwigshafen (DE)
(74) Vertreter: Schuck, Alexander

(57) **Zusammenfassung**

Verfahren zur Herstellung von Vinylalkylenglykolethern mit den Schritten:
(i) Bereitstellung von einem oder mehreren gegebenenfalls substituierten Phenylalkylenglykolethern der allgemeinen Formel (I), 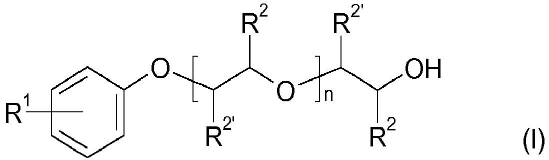 worin R¹ Cl oder NO₂ und R² und R^{2'} unabhängig voneinander H oder C₁-C₃-Alkyl bedeuten und n = 1 bis 155 ist,
(ii) Eliminierung von gegebenenfalls substituiertem Phenol aus dem oder den Phenylalkylenglykolethern der allgemeinen Formel (I) in Gegenwart einer Base, wobei ein oder mehrere Vinyalkylenglykolether der allgemeinen Formel (II) 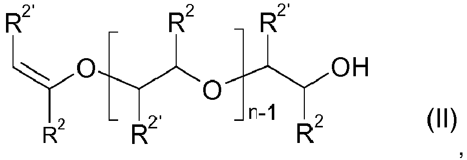 worin R², R^{2'} und n die oben angegebene Bedeutung haben,
erhalten werden.

## Beschreibung

Die Erfindung betrifft ein Verfahren zur Herstellung von Vinylalkylenglykolethern.

Vinyletheralkohole wie Vinyldiethylenglykolether sind wertvolle Ausgangsmaterialien zur Herstellung von Vinylpolyethylenglykol (Vinyl-PEG), welches als Makromonomer für die Herstellung von Betonverflüssigern verwendet werden kann.

Vinylether werden nach dem Reppe-Verfahren durch Umsetzung von Hydroxyverbindungen mit Acetylen hergestellt. Die Umsetzung erfolgt in Gegenwart einer Base, beispielsweise dem Alkoholat der Hydroxyverbindung, welches durch Zugabe von Alkalimetallhydroxid zu der Hydroxyverbindung gebildet wird.

WO 2012/110437 offenbart ein Verfahren zur Herstellung von Divinylethern durch Umsetzung von aliphatischen Diolen, vorzugsweise Diethylenglykol oder Triethylengykol, mit Acetylen, bei dem keine vollständige Umsetzung der Hydroxylgruppen mit Acetylen erfolgt und das erhaltene Produktgemisch neben dem Divinylether noch den Monovinylether enthält und der Monovinylether durch eine Extraktivdestillation in Gegenwart eines Extraktionsmittels aus dem Produktgemisch abgetrennt wird.

Ein Nachteil der Herstellung von Vinylethylenglykolethern durch Umsetzung von Diolen mit Acetylen nach dem Reppe-Verfahren ist, dass im Allgemeinen ein Gemisch aus Monovinylethern und Divinylethern anfällt. Acetylen ist eine hochentzündliche Substanz, die nur unter strengsten Sicherheitsvorkehrungen gehandhabt werden kann. Sie kann in Gegenwart von Zündquellen aller Art detonativ in die Elemente zerfallen, wobei große Energiemengen frei werden. Diese Eigenschaften machen Prozesse, in denen Acetylen eingesetzt wird, aufwändig und kostspielig.

Aufgabe der Erfindung ist es, ein Alternativverfahren zur Herstellung von Vinylethylenglykolethern und allgemein Vinylalkylenglykolethern bereitzustellen. Insbesondere ist es Aufgabe der Erfindung, ein verbessertes Verfahren zur Herstellung von Vinylalkylenglykolethern bereitzustellen, bei dem ausschließlich die Monovinylether gebildet werden.

Gelöst wird die Aufgabe durch ein Verfahren zur Herstellung von Vinylalkylenglykolethern mit den Schritten:
(i) Bereitstellung von einem oder mehreren gegebenenfalls mit R¹ ein- bis dreifach substituierten Phenylalkylenglykolethern der allgemeinen Formel (I), 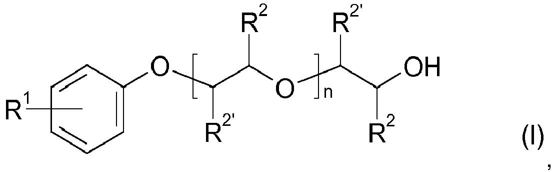 worin R¹ unabhängig voneinander Cl oder NO₂ bedeutet, R² und R^{2'} unabhängig voneinander H oder C₁-C₃-Alkyl bedeuten und n = 1 bis 155 ist,
(ii) Eliminierung von gegebenenfalls substituiertem Phenol aus dem oder den Phenylalkylenglykolethern der allgemeinen Formel (I) in Gegenwart einer Base, wobei ein oder mehrere Vinylalkylenglykolether der allgemeinen Formel (II) erhalten werden 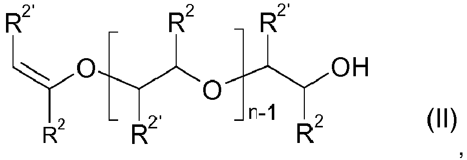 worin R² und R^{2'} unabhängig voneinander H oder C₁-C₃-Alkyl bedeuten und n =1 bis 155 ist.

Die Phenylalkylenglykolether der allgemeinen Formel (I), von denen das erfindungsgemäße Verfahren ausgeht, enthalten 1 bis 155 Alkylenoxid-Wiederholungseinheiten (n = 1 bis 155). Diese können durch Alkoxilierung von unsubstituiertem bzw. substituiertem Phenol erhalten werden.

Im Allgemeinen ist in jeder der Alkylenoxid-Wiederholungseinheiten nur höchstens einer der Reste R² und R^{2'} C₁-C₃-Alkyl und der andere Wasserstoff.

Vorzugsweise werden Phenylethylenglykolether umgesetzt zu Vinylethylenglykolethern, d.h. R² und R^{2'} in Formel (I) sind Wasserstoff.

Besonders bevorzugt werden unsubstituierte Phenylethylenglykolether der allgemeinen Formel (la) umgesetzt, d.h. R¹, R² und R^{2'} in Formel (I) sind Wasserstoff: 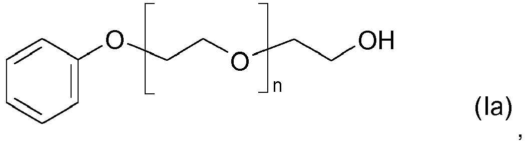 worin n=1 bis 155 ist.

In einer Ausführungsform wird als Phenylalkylenglykolether der allgemeinen Formel (la) Phenyldiethylenglykolether (n = 1) eingesetzt. Phenyldiethylenglykolether entsteht als Nebenprodukt bei der Herstellung von Phenoxyethanol. Phenoxyethanol ist ein gängiges Biozid und wird in großen Mengen hergestellt.

In einer weiteren Ausführungsform werden Gemische enthaltend Phenyldiethylenglykolether und Phenyltriethylenglykolether (n = 1, 2) eingesetzt. Derartige Gemische fallen an bei der Umsetzung von Phenoxyethanol mit Ethylenoxid nach einer partiellen Aufreinigung durch Destillation, da die Produktverteilung bei der Ethoxylierung von Alkoholen einer Gauß-Verteilung nahekommt.

In einer weiteren Ausführungsform werden Gemische enthaltend Phenylethylenglykolether der allgemeinen Formel (Ia) mit n = 1 bis 5 eingesetzt. Derartige Gemische fallen ebenso bei der Ethoxylierung von Phenoxyethanol an, wenn anschließend keine fraktionierende Destillation durchgeführt wird.

Die Eliminierungsreaktion (ii) erfolgt in Gegenwart einer Base. Geeignete Basen sind die Hydroxide, Carbonate, Hydogencarbonate, Hydride und Alkoholate von Alkali- und Erdalkalimetallen. Bevorzugte Basen sind Alkalimetallhydroxide (außer Lithiumhydroxid) wie Natriumhydroxid oder Kaliumhydroxid und Alkalimetallalkoholate wie Natriummethylat und Kaliummethylat.

Die Phenylalkylenglykolether (I) können in einem separaten Lösungsmittel vorliegen. Geeignete Lösungsmittel sind polare Verbindungen, die höher sieden als die gewünschten Vinylether, wie Tetraethylenglykoldimethylether (TEGDME), Diethylenglykoldimethylether (Diglyme), andere hochsiedende Ether, Alkohole wie Hexanol, Octanol, Dodecanol, Isoamylalkohol, Triethylenglykol, Amide wie N-Methylpyrrolidon, N-Formylmorpholin und Dimethylacetamid, Sulfoxide wie Dimethylsulfoxid, Harnstoffe wie Dimethylpropylenharnstoff und Dimethylethylenharnstoff.

Die Eliminierungsreaktion kann auch in Abwesenheit eines separaten Lösungsmittels durchgeführt werden.

Im Allgemeinen wird die Eliminierungsreaktion durch Erhitzen der Phenylalkylenglykolether (I), die gegebenenfalls in einem separaten Lösungsmittel vorliegen, in Gegenwart einer Base auf eine Temperatur im Bereich von 100 bis 300°C, vorzugsweise 130 bis 260°C durchgeführt. Die Base kann den Phenylalkylenglykolethern (I) oder einer die Phenylalkylenglykolether (I) enthaltenden Lösung als Feststoff zugegeben werden.

Bei der Eliminierungsreaktion wird durch die Base Wasserstoff abstrahiert und Phenol in Form von Alkalimetall- oder Erdalkalimetallphenolat eliminiert, wodurch eine Vinylgruppe gebildet wird.

In einer Ausführungsform der Erfindung wird die Eliminierungsreaktion (ii) in Gegenwart eines Kronenethers, beispielsweise 18-Krone-6, durchgeführt. Durch die Anwesenheit eines Kronenethers kann die Basenstärke des basischen Anions erhöht werden, was sich günstig auf die Eliminierungsreaktion auswirken kann.

In einer weiteren Ausführungsform der Erfindung wird die Eliminierungsreaktion in Gegenwart von Tetraethylenglykoldimethylether (TEGDME) durchgeführt. Dieses fungiert einerseits als Lösungsmittel, andererseits als Komplexierungsmittel für Alkaliionen, insbesondere Kalium, wodurch die Basenstärke des basischen Anions erhöht wird, was sich günstig auf die Eliminierungsreaktion auswirken kann.

Die Abspaltung von Phenolat wird im Allgemeinen unter einer inerten Schutzgasatmosphäre unter Ausschluss von Sauerstoff durchgeführt. Geeignete Schutzgase sind zum Beispiel Stickstoff und Argon. Sie kann auch unter Schutzgas bei Normaldruck oder erhöhtem Druck oder unter reduziertem Druck nach dem Prinzip einer Reaktivdestillation durchgeführt werden. Dabei wird in den Sumpf der Reaktionskolonne Energie eingetragen, was die Abspaltungsreaktion befördert. Der entstehende Vinylether wird als Leichtsieder diesem Reaktionsgemisch kontinuierlich entzogen und am Kopf der Kolonne auskondensiert und abgeleitet. Es ist auch möglich, inertes Gas dem Sumpf der Kolonne zuzuführen und mit dem Gasstrom den Vinylether aus der Reaktionskolonne auszuschleusen. Dieses Prinzip ist auch bei der Durchführung der Reaktion in einem herkömmlichen Reaktionskessel mit einer aufgesetzten Destillationskolonne oder in einer Blasensäule einsetzbar.

Aus dem Reaktionsgemisch, das noch nicht umgesetzte Phenylalkylenglykolether enthalten kann, können die einzelnen Vinylalkylenglykolether durch Destillation isoliert werden.

Die erhaltenen Vinylalkylenglykolether der allgemeinen Formel (II) können zu höhermolekularen Vinylethylenglykolethern ethoxiliert und allgemein zu Vinylalkylenglykolethern alkoxiliert werden. Dabei werden die Vinyletheralkohole in Gegenwart eines basischen Katalysators oder eines Doppelmetallcyanid-Katalysators mit Alkylenoxiden bei Temperaturen von 50 bis 180°C umgesetzt.

Als Katalysatoren können basische Katalysatoren oder Doppelmetallcyanide eingesetzt werden. Beispiele für Doppelmetallcyanide sind Zinkhexacyanocobaltat(III), Zinkhexacyanoferrat(III), Zinkhexacyanoferrat(II), Nickel(II)hexacyanoferrat(II), Cobalt(II)hexacyanocobaltat(III) und ähnliches. Weitere Beispiele für geeignete Doppelmetallcyanide sind in US 5,158,922 aufgeführt.

Geeignete anorganische Basen sind zum Beispiel Metallsalze anorganischer Säuren oder Metalloxide oder -hydroxide oder -hydride sowie die Alkalimetalle selbst. In einer bevorzugten Ausführungsform werden Metallhydroxide verwendet. Bevorzugt sind weiterhin Salze anorganischer Säuren mit Alkali- oder Erdalkalimetallen. Besonders bevorzugt sind Hydroxide von Alkali- oder Erdalkalimetallen. Ganz Besonders bevorzugt sind Natriumhydroxid, Kaliumhydroxid oder Calciumhydroxid. Insbesondere bevorzugt ist Kaliumhydroxid.

In einer weiteren bevorzugten Ausführungsform werden metallorganische Verbindungen eingesetzt. In einer weiteren bevorzugten Ausführungsform ist der Katalysator ein Metallalkoholat. Bevorzugt sind Alkoholate von Alkali- oder Erdalkalimetallen, besonders bevorzugt sind Alkoholate von Natrium oder Kalium. Weiterhin sind Alkoholate von aliphatischen oder cycloaliphatischen Alkoholen bevorzugt. Besonders bevorzugt sind Alkoholate von linearen oder verzweigten Alkanolen, insbesondere solche von C₁- bis C₅-Alkanolen. Ganz besonders bevorzugt sind Methanolate und Ethanolate. Insbesondere bevorzugt sind Natriummethanolat und Kaliummethanolat. Es ist ebenfalls möglich, Gemische verschiedener Katalysatoren einzusetzen.

Der Katalysator wird im Allgemeinen in Wasser oder Alkohol oder in Lösemittelgemischen, die Wasser und/oder mindestens einen Alkohol enthalten, gelöst eingesetzt. Dabei kann der Anteil an Wasser und Alkohol in weiten Grenzen variieren. Bevorzugt beträgt der Anteil an Wasser und/oder Alkohol am Lösemittelgemisch mindestens 10 %, besonders bevorzugt mindestens 50 %, ganz besonders bevorzugt mindestens 80 %. Insbesondere bevorzugt besteht das Lösemittel für den Katalysator zu mindestens 80 % aus Wasser oder einem Alkohol. Bevorzugte Alkohole sind lineare oder verzweigte Alkanole, besonders bevorzugt mit einem bis fünf Kohlenstoffatomen. Ist die Base ein Alkoholat, so wird als Lösungsmittel zweckmäßigerweise bevorzugt der korrespondierende Alkohol verwendet. Besonders bevorzugt sind Lösungen von Natriumhydroxid
oder Kaliumhydroxid in Wasser oder Lösungen von Natriummethanolat in Methanol.

Bevorzugt handelt es sich um Lösungen mit einem Gehalt an Base von 1 bis 80 Gew.-%, bevorzugt von 5 bis 70 Gew.-%, ganz besonders bevorzugt von 10 bis 60 Gew.-% und insbesondere bevorzugt von 30 bis 50 Gew.-%.

Nach der Zugabe des Katalysators ist im Allgemeinen ein Destillationsschritt erforderlich. Dieser Destillationsschritt entfällt bei der Verwendung von Basen wie metallischem Alkalimetall oder Alkalimetallhydrid und Alkalimetallorganylen wie Butyllithium, da in diesen Fällen Wasserstoff oder ein Kohlenwasserstoff entsteht.

Der Destillationsschritt erfolgt vor der Zugabe von Alkylenoxid und wird bevorzugt bei Destillationstemperaturen von 80 bis 145°C, besonders bevorzugt bei 120 bis 135°C durchgeführt. Üblicherweise wird ein Destillationsvakuum von 10 bis 500 mbar, bevorzugt von 60 bis 200 mbar, besonders bevorzugt von 150 bis 170 mbar angelegt. In einer bevorzugten Ausführungsform der Erfindung wird der Destillationsschritt derart ausgeführt, dass Vinylalkylenglykolether (II) und Katalysator bei gleichzeitiger Evakuierung unter Destillationsvakuum aufgeheizt werden. Bei Erreichen der Destillationstemperatur wird das Gemisch für eine gewisse Zeitspanne den Destillationsbedingungen unterworfen. Diese Zeitspanne kann kurz sein, zum Beispiel unterhalb einer Stunde liegen.

Nach Beendigung der Destillation ist das Starter/Katalysatorgemisch im Wesentlichen frei von Wasser oder den im Katalysatorgemisch enthaltenen Alkoholen und den korrespondierenden Alkoholen der Alkoholate. Typischerweise enthält dieses dann weniger als 1000 ppm Wasser.

In einigen Fällen ist nach der Destillation noch eine größere Menge an Wasser enthalten, zum Beispiel bis zu 5000 ppm. Bevorzugt wird die Destillation jedoch so ausgeführt, dass sogar weniger als 500 ppm oder sogar weniger als 200 ppm Wasser in der Mischung enthalten sind.

Die Zugabe von Alkylenoxid erfolgt vorteilhaft nach beendeter Destillation. Anschließend wird Alkylenoxid mit einem Wassergehalt von im Allgemeinen maximal 100 ppm, bevorzugt maximal 50 ppm zudosiert. Die Alkylenoxide reagieren mit dem vorgelegten Starter. Sind die eingesetzten Alkylenoxide unter Normalbedingungen gasförmig, geschieht die Zugabe zweckmäßigerweise unter erhöhtem Druck. Bevorzugt erfolgt die Zugabe von Alkylenoxid über einen längeren Zeitraum, zum Beispiel über mehrere Stunden, verteilt. Die Alkoxylierung wird vorzugsweise bei einer Temperatur von maximal 150°C, besonders bevorzugt von 125 bis 145°C durchgeführt. Bei dieser Temperatur kommt es nicht in nennenswertem Ausmaß zu Eliminierungsreaktionen und Kettenabbruch. Die Reaktion wird im Allgemeinen in einem Druckreaktor durchgeführt. Der Alkylenoxid-Druck beträgt dabei im Allgemeinen 1 bis 20 bar, bevorzugt 1 bis 6 bar.

Die Zugabe kann dabei unterbrochen und anschließend wieder aufgenommen werden. Werden verschiedene Alkylenoxide eingesetzt, so können diese als Gemisch zugegeben werden oder nacheinander. Nachdem die gesamte Menge an Alkylenoxid zugegeben worden ist, kann das Reaktionsgemisch noch bis zur Druckkonstanz bei Reaktionstemperatur gerührt werden, um einen möglichst vollständigen Umsatz des Alkylenoxids zu gewährleisten. Restmengen von nicht umgesetztem Alkylenoxid können durch Anlegen von Vakuum oder durch Strippen mit einem Inertgas aus dem Reaktionsgemisch entfernt werden. Der Druckreaktor wird abgekühlt und entspannt. Das Produktgemisch wird im Allgemeinen durch Zugabe einer Säure, beispielsweise Essigsäure, Phosphorsäure oder CO₂ neutralisiert. Vorteilhafterweise wird die Neutralisation bei <100°C durchgeführt.

Optional können die Vinylpolyalkylenglykole entsalzt werden, z.B. durch Ausfällen von Kalium und Natrium mit Magnesiumsilikat, Phosphorsäure, Polyphosphorsäure oder einem alternativen Fällungsmittel. Ebenso kann das Reaktionsprodukt mittels lonentauscher entsalzt werden.

Die Erfindung wird durch die nachstehenden Beispiele näher erläutert.

### Beispiele

Die Analytik wurde mittels Gaschromatographie durchgeführt. Die verwendete Säule war eine RTX5 Amin 30 m, 0,32 mm, 1,5 µm. Temperaturprogramm: 80°C bis 280°C mit 10°C/min. Alle Angaben erfolgen in GC-Flächen-%.

### Beispiel 1

In einen 100 ml-Dreihalskolben mit Magnetrührer, Destillationsbrücke, Stickstoffleitung, Blasenzähler und Innenthermometer wurden 75,1 g Phenylethylenglykol in GC-Flächenprozent (0,6 % Phenoxyethanol, 11,9 % Diethylenglykolmonophenylether, 24,2 % Triethylenglykolmonophenylether, 25,2 % Tetraethylenglykolmonophenylether, 19,6 % Pentaethylenglykolmonophenylether, 14 % Hexaethylenglykolmonophenylether, 4 % sonstige) und 7,5 g festes Natriumhydroxid als Plätzchen zugegeben. Der Kolben wurde mit Stickstoff inertisiert und in einen Heizpilz der Marke Horst Laborgerätematerial GmbH gestellt und auf 160 bis 165°C (laut Innentemperaturmessung) erwärmt. Beim Erwärmen löste sich das NaOH vollständig. Nach 3 h bei 160 bis 165°C wurde abgekühlt, eine Probe mit Methanol verdünnt und per GC analysiert. Das Reaktionsgemisch enthielt (alle Angaben in GC-Flächenprozent) 0,1 % Phenol, 1,5 % Vinyldiethylenglykolether, 1,8 % Vinyltriethylenglykolether, 2,5 % Vinyltetraethylenglykolether, 1,5 % Vinylpentaethylenglykolether, 0,5 % Phenoxyethanol, 14,5 % Diethylenglykolmonophenylether, 29 % Triethylenglykolmonophenylether, 25,2 % Tetraethylenglykolmonophenylether, 27,8 % Pentaethylenglykolmonophenylether, 2,5 % Hexaethylenglykolmonophenylether und 7,4 % sonstige, nicht zugeordnete Verbindungen.

### Beispiel 2

Die verwendete Apparatur und das Edukt entsprachen denen aus Beispiel 1. Anstelle von NaOH wurde festes KOH in Form von Plätzchen eingesetzt. Das Reaktionsgemisch enthielt 1,1 % Phenol, 1,7 % Vinyldiethylenglykolether, 2,1 % Vinyltriethylenglykolether, 1,8 % Vinyltetraethylenglykolether, 2,2 % Vinylpentaethylenglykolether, 0,4 % Phenoxyethanol, 9,9 % Diethylenglykolmonophenylether, 21,6 % Triethylenglykolmonophenylether, 26,6 % Tetraethylenglykolmonophenylether, 20,0 % Pentaethylenglykolmonophenylether, 6,7 % Hexaethylenglykolmonophenylether und 5,9 % sonstige, nicht zugeordnete Verbindungen.

### Vergleichsbeispiel 1

Die verwendete Apparatur und das Edukt entsprachen denen aus Beispiel 1. Anstelle von NaOH wurde festes LiOH-Pulver eingesetzt. Das Reaktionsgemisch enthielt 0,0 % Vinylether.

### Vergleichsbeispiel 2

Die verwendete Apparatur und das Edukt entsprachen denen aus Beispiel 1. Anstelle von NaOH wurde kein Zusatz eingesetzt. Das Reaktionsgemisch enthielt 0,0 % Vinylether.

### Beispiel 3

Die verwendete Apparatur und das Edukt entsprachen denen aus Beispiel 1. Anstelle von NaOH wurde festes KOH in Form von Plätzchen eingesetzt. Zusätzlich wurde die Temperatur auf 180°C eingestellt. Das Reaktionsgemisch enthielt 2,6 % Phenol, 2,4 % Vinyldiethylenglykolether, 2,5 % Vinyltriethylenglykolether, 2,2 % Vinyltetraethylenglykolether, 2,4 % Vinylpentaethylenglykolether, 0,4 % Phenoxyethanol, 9,0 % Diethylenglykolmonophenylether, 19,6 % Triethylenglykolmonophenylether, 24,8 % Tetraethylenglykolmonophenylether, 19,2 % Pentaethylenglykolmonophenylether, 6,7 % Hexaethylenglykolmonophenylether und 8,2 % sonstige, nicht zugeordnete Verbindungen.

### Beispiel 4

Die verwendete Apparatur und das Edukt entsprachen denen aus Beispiel 1. Anstelle von NaOH wurde festes KOH in Form von Plätzchen eingesetzt. Zusätzlich wurde die Temperatur auf 180°C eingestellt und es wurde noch 0,4 g Kronenether 18-6 zugegeben. Das Reaktionsgemisch enthielt 2,8 % Phenol, 3,3 % Vinyldiethylenglykolether, 2,9 % Vinyltriethylenglykolether, 3,1 % Vinyltetraethylenglykolether, 4,1 % Vinylpentaethylenglykolether, 0,4 % Phenoxyethanol, 10,4 % Diethylenglykolmonophenylether, 22,4 % Triethylenglykolmonophenylether, 23,3 % Tetraethylenglykolmonophenylether, 15,2 % Pentaethylenglykolmonophenylether, 4,3 % Hexaethylenglykolmonophenylether und 8,0 % sonstige, nicht zugeordnete Verbindungen.

### Beispiel 5

Die verwendete Apparatur und das Edukt entsprachen denen aus Beispiel 1. Anstelle von NaOH wurde festes KOH in Form von Plätzchen eingesetzt. Zusätzlich wurde die Temperatur auf 180°C eingestellt und es wurden noch 37,6 g Tetraethylenglykoldimethylether zugegeben. Das Reaktionsgemisch enthielt 6,0 % Phenol, 5,3 % Vinyldiethylenglykolether, 3,9 % Vinyltriethylenglykolether, 3,5 % Vinyltetraethylenglykolether, 3,2 % Vinylpentaethylenglykolether, 0,7 % Phenoxyethanol, 11,2 % Diethylenglykolmonophenylether, 22,6 % Triethylenglykolmonophenylether, 22,1 % Tetraethylenglykolmonophenylether, 11,6 % Pentaethylenglykolmonophenylether, 2,9 % Hexaethylenglykolmonophenylether, 7,0 % sonstige, nicht zugeordnete Verbindungen.

Nach der Entnahme der oben beschriebenen Probe wurde die Temperatur auf 260°C erhöht und 2 h bei dieser Temperatur gehalten. Das Reaktionsgemisch enthielt 14,8 % Phenol, 5,1 % Vinyldiethylenglykolether, 5,3 % Vinyltriethylenglykolether, 4,6 % Vinyltetraethylenglykolether, 3,3 % Vinylpentaethylenglykolether, 1,6 % Phenoxyethanol, 8,8 % Diethylenglykolmonophenylether, 18,1 % Triethylenglykolmonophenylether, 16,4 % Tetraethylenglykolmonophenylether, 8,5 % Pentaethylenglykolmonophenylether, 2,1 % Hexaethylenglykolmonophenylether und 11,9 % sonstige, nicht zugeordnete Verbindungen.

### Vergleichsbeispiel 3

Die verwendete Apparatur entsprach der aus Beispiel 1,75 g Diethylenglykolmonophenylether wurden mit 5 mol-% para-Toluensulfonsäure versetzt und 6 h unter Stickstoff bei 160°C gerührt. Das Reaktionsgemisch wurde per Gaschromatographie analysiert. Es enthielt keine Vinyletherverbindungen. Das Edukt war zu ca. 19 % umgesetzt, es hatte sich hauptsächlich zu Phenol (2,3 %) und dem Tetraethylenglykoldiphenylether (8,2 %) umgesetzt. Daneben wurden Spuren von Monoethylenglykol und Triethylenglykoldiphenylether sowie 5,4 % sonstige, nicht zugeordnete Verbindungen gefunden.

### Beispiel 7

Die verwendete Apparatur entsprach der aus Beispiel 1. 168,1 g Diethylenglykolmonophenylether wurden mit 16,8 g festem KOH in Form von Plätzchen versetzt und 5 h bei 200°C und 2 h bei 210°C unter einem leichten Stickstoffstrom gerührt. Dabei wurden 28,1 g Destillat in einem Kolben hinter der Destillationsbrücke aufgefangen. Dieses Destillat wurde per GC analysiert und enthielt 50,3 % Methyldioxolan, 0,7 % Monoethylenglykol, 8,4 % Vinyloxyethanol, 0,6 % Phenol, 15,6 % Diethylenglykolmonophenylether, 0,3 % Vinyloxyethoxyethanol und 6,2 % sonstige, nicht zugeordnete Verbindungen.

### Beispiel 8

In einen 50 ml-Dreihalskolben mit Magnetrührer, Destillationsbrücke, Stickstoffleitung, Blasenzähler und Innenthermometer wurden 7,5 g eines Gemisches von Diethylenglykolmonophenylether (56,3 %), Triethylenglykolmonophenylether (41,6 %) und höheren Ethern (1,8 %) mit 0,94 g KOCH₃ in Form von Pulver mit 1,9 g Tetraethylenglykoldimethylether (TEGDME) zusammengegeben und in leichtem N₂-Strom 30 Minuten bei 150°C gerührt. Das Reaktionsgemisch wurde per GC analysiert. Das Gemisch enthielt 6,5 % Vinyloxyethoxyethanol, 0,2 % Phenoxyethanol und 4,6% sonstige, nicht zugeordnete Verbindungen.

### Beispiel 9

In der in Beispiel 8 beschriebenen Apparatur wurden 7,5 g eines Gemisches von Diethylenglykolmonophenylether (56,3 %), Triethylenglykolmonophenylether (41,6 %) und höheren Ethern (1,8 %) mit 4,54 g KOH in Form von Plätzchen sowie 1,9 g TEGDME versetzt. Dies entsprach ungefähr 1,8 molaren Äquivalenten, bezogen auf das Phenylethergemisch mit einer gemittelten Molmasse von 195 g/mol. Alles zusammen wurde 30 Minuten bei 130°C gerührt und das hellbraune Reaktionsgemisch dann per GC analysiert. Es enthielt 1,7 % Methyldioxolan, 10,8 % Vinyloxyethanol, 0,3 % Phenol, 16,0 % Vinyloxyethoxyethanol und 14,7 % sonstige, nicht zugeordnete Verbindungen. Die Differenz zu 100 % bestand aus TEGDME und Edukten.

### Beispiel 10

In der in Beispiel 8 beschriebenen Apparatur wurden 2,0 g eines Gemisches von Diethylenglykolmonophenylether (56,3 %), Triethylenglykolmonophenylether (41,6 %) und höheren Ethern (1,8 %) mit 1,1 g KOH (entsprechend ungefähr 1,6 molaren Äquivalenten, bezogen auf die Phenylether mit einer gemittelten Molmasse von 195 g/mol) in Form von Plätzchen sowie mit 0,5 g TEGDME versetzt. Dazu wurden noch 15 g Wasser gegeben und das Gemisch dann 3 h lang bei 100°C gerührt. Das farblose Gemisch wurde dann per GC analysiert. Es enthielt 2,8 % Vinyloxyethanol, 0,7 % Phenol, 6,1 % Vinyloxyethoxyethanol und 11,4 % sonstige, nicht zugeordnete Verbindungen. Die Differenz zu 100 % bestand aus TEGDME und Edukten

### Beispiel 11

In der in Beispiel 1 beschriebenen Apparatur wurden 25,0 g eines Gemisches von Diethylenglykolmonophenylether (1,9 %), Triethylenglykolmonophenylether (89,9 %) und höheren Ethern (8,2 %) mit 13,4 g KOH (entsprechend ungefähr 2,0 molaren Äquivalenten bezogen auf die Phenylether mit einer gemittelten Molmasse von 229 g/mol) in Form von Plätzchen sowie mit 25 g TEGDME versetzt. Das Gemisch wurde 4 h bei 150°C und 0,5 h bei 180°C unter Stickstoff gerührt. Es wurden 59 g isoliert. Das Gemisch wurde per Gaschromatographie analysiert. Es enthielt 0,1 % Phenol, 18,1 % Vinyloxyethoxyethanol und 11,6 % nicht umgesetzte Phenylether, 63,1 % TEGDME und 6,9 % sonstige, nicht zugeordnete Verbindungen. Dieselbe Probe wurde mit einer Methode analysiert, bei der durch Kalibration mit Eichlösungen unterschiedlichen Gehalts an Vinyloxyethoxyethanol der Gehalt an Vinyloxyethoxyethanol quantitativ erfasst wurde. Dieser betrug 11,8 Gew.-%. Dies entspricht einer Masse von 7,0 g Vinyloxyethoxyethanol im Reaktionsgemisch und einer Ausbeute von 53 %.

### Beispiel 12

In der in Beispiel 1 beschriebenen Apparatur wurden 25,0 g eines Gemisches von Diethylenglykolmonophenylether (1,9 %), Triethylenglykolmonophenylether (89,9 %) und höheren Ethern (8,2 %) mit 13,4 g KOH (entsprechend ungefähr 2,0 molaren Äquivalenten bezogen auf die Phenylether mit einer gemittelten Molmasse von 229 g/mol) in Form von Plätzchen sowie 25 g TEGDME versetzt. Das Gemisch wurde 4 h bei 150°C und 0,5 h bei 180°C unter Stickstoff gerührt. Es wurden 59 g isoliert. Das Gemisch wurde per Gaschromatographie analysiert. Es enthielt 0,1 % Phenol, 18,1 % Vinyloxyethoxyethanol und 11,6 % nicht umgesetzte Phenylether, 63,1 % TEGDME und 6,9 % sonstige, nicht zugeordnete Verbindungen. Dieselbe Probe wurde mit einer Methode analysiert, bei der durch Kalibration mit Eichlösungen unterschiedlichen Gehalts an Vinyloxyethoxyethanol der Gehalt an Vinyloxyethoxyethanol quantitativ erfasst wurde. Dieser betrug 11,8 Gew.-%. Dies entspricht einer Masse von 7,0 g Vinyloxyethoxyethanol im Reaktionsgemisch und einer Ausbeute von 53 %.

## Patentansprüche

1. Verfahren zur Herstellung von Vinylalkylenglykolethern mit den Schritten:
(i) Bereitstellung von einem oder mehreren gegebenenfalls substituierten Phenylalkylenglykolethern der allgemeinen Formel (I), 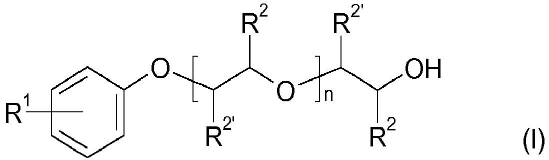 worin R¹ Cl oder NO₂ und R² und R^{2'} unabhängig voneinander H oder C₁-C₃-Alkyl bedeuten und n = 1 bis 155 ist,
(ii) Eliminierung von gegebenenfalls substituiertem Phenol aus dem oder den Phenylalkylenglykolethern der allgemeinen Formel (I) in Gegenwart einer Base, wobei ein oder mehrere Vinyalkylenglykolether der allgemeinen Formel (II), 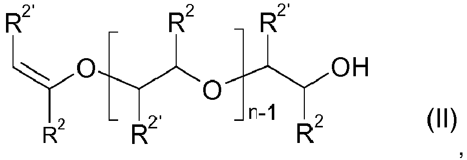 worin R², R^{2'} und n die oben angegebene Bedeutung haben,
erhalten werden.

2. Verfahren nach Anspruch 1, **dadurch gekennzeichnet, dass** in Schritt (i) unsubstituierte Phenylethylenglykolether der allgemeinen Formel (la) bereitgestellt werden

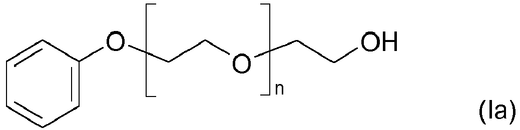

3. Verfahren nach Anspruch 2, **dadurch gekennzeichnet, dass** in Schritt (i) ein Gemisch aus Phenylethylenglykolethern der Formel (la) mit n = 1 bis 5 bereitgestellt wird.

4. Verfahren nach Anspruch 2, **dadurch gekennzeichnet, dass** in Schritt (i) ein Gemisch enthaltend Phenyldiethylenglykolether und Phenyltriethylenglykolether bereitgestellt wird.

5. Verfahren nach Anspruch 2, **dadurch gekennzeichnet, dass** in Schritt (i) ein Phenyldiethylenglykolether, der als Nebenprodukt bei der Herstellung von Phenoxyethanol erhalten wird, bereitgestellt wird.

6. Verfahren nach einem der Ansprüche 1 bis 5, **dadurch gekennzeichnet, dass** Schritt (ii) in Gegenwart von Alkalimetallhydroxid als Base durchgeführt wird.

7. Verfahren nach einem der Ansprüche 1 bis 6, **dadurch gekennzeichnet, dass** Schritt (ii) bei einer Temperatur von 100 bis 300°C durchgeführt wird.

8. Verfahren nach einem der Ansprüche 1 bis 7, **dadurch gekennzeichnet, dass** Schritt (ii) in einem separaten Lösungsmittel durchgeführt wird.

9. Verfahren nach einem der Ansprüche 1 bis 7, **dadurch gekennzeichnet, dass** Schritt (ii) in Abwesenheit eines separaten Lösungsmittels durchgeführt wird.

10. Verfahren nach einem der Ansprüche 1 bis 9, **dadurch gekennzeichnet, dass** Schritt (ii) in Gegenwart eines Kronenethers durchgeführt wird.

11. Verfahren nach einem der Ansprüche 1 bis 9, **dadurch gekennzeichnet, dass** Schritt (ii) in Gegenwart von Tetraethylenglykoldimethylether durchgeführt wird.
